(19) Europäisches Patentamt
European Patent Office
Office européen des brevets

(11) **EP 2 906 949 B1**

(12) **EUROPÄISCHE PATENTSCHRIFT**

(45) Veröffentlichungstag und Bekanntmachung des Hinweises auf die Patenterteilung:
**23.08.2017 Patentblatt 2017/34**

(21) Anmeldenummer: **13774187.2**

(22) Anmeldetag: **09.10.2013**

(51) Int Cl.:
**G01N 33/543** (2006.01)    **G01R 33/00** (2006.01)

(86) Internationale Anmeldenummer:
**PCT/EP2013/071066**

(87) Internationale Veröffentlichungsnummer:
**WO 2014/056987 (17.04.2014 Gazette 2014/16)**

(54) **NACHWEIS EINES ANALYTEN UND BESTIMMUNG DER KONZENTRATION EINES ANALYTEN MIT HILFE VON MAGNETISIERBAREN BEADS**

DETECTION OF AN ANALYTE AND DETERMINING THE CONCENTRATION OF AN ANALYTE BY MEANS OF MAGNETISABLE BEADS

CONTRÔLE D'UN ANALYTE ET DÉTERMINATION DE LA CONCENTRATION D'UN ANALYTE À L'AIDE DE BILLES MAGNÉTISABLES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.10.2012 EP 12188204**
**13.11.2012 EP 12192353**

(43) Veröffentlichungstag der Anmeldung:
**19.08.2015 Patentblatt 2015/34**

(73) Patentinhaber: **Orgentec Diagnostika GmbH**
**55129 Mainz (DE)**

(72) Erfinder:
• **KIRSTE, Vinzenz**
**55270 Zornheim (DE)**
• **BERG, Wigbert**
**55131 Mainz (DE)**
• **SOSKIC, Vukic**
**55131 Mainz (DE)**

(74) Vertreter: **Weickmann & Weickmann PartmbB**
**Postfach 860 820**
**81635 München (DE)**

(56) Entgegenhaltungen:
**US-B1- 6 294 342**

• **E. RAPOPORT ET AL: "Integrated capture, transport, and magneto-mechanical resonant sensing of superparamagnetic microbeads using magnetic domain walls", LAB ON A CHIP, Bd. 12, Nr. 21, 1. Januar 2012 (2012-01-01), Seite 4433, XP055046582, ISSN: 1473-0197, DOI: 10.1039/c2lc40715a**
• **WEBER W ET AL: "Magnet-guided transduction of mammalian cells and mice using engineered magnetic lentiviral particles", JOURNAL OF BIOTECHNOLOGY, ELSEVIER SCIENCE PUBLISHERS, AMSTERDAM, NL, Bd. 141, Nr. 3-4, 20. Mai 2009 (2009-05-20), Seiten 118-122, XP026099295, ISSN: 0168-1656, DOI: 10.1016/J.JBIOTEC.2009.02.023 [gefunden am 2009-03-17]**

**Beschreibung**

Die Erfindung betrifft Verfahren zum Nachweis eines Analyten in einer Probe.

Für den Nachweis von Analyten, beispielsweise klinischer Parameter für die medizinische Diagnostik, gibt es viele unterschiedliche Verfahren, bei denen das Vorhandensein und/oder die Konzentration des Analyten in einer Probe mittels geeigneter Methoden, insbesondere optischer und/oder elektrochemischer Methoden, bestimmt wird.

Das US-Patent US 6,294,342 B1 beschreibt die Messung von Komplexen aus magnetischen Beads, die mittels Analyt an ein mobiles Festphasenreagenz gebunden sind. Bei der Messung wird die Kraft, welche magnetisierbare Partikel auf einen Magneten ausüben, gemessen, wobei sich der Magnet unter dem Probengefäß befindet. Durch die Anziehung des Magneten bewegen sich die Komplexe auf diesen zu und diese Bewegung in Richtung des Magneten kann mittels eines Hall-Effekt-Sensors gemessen werden. Eine Problem der beschriebenen Konfiguration besteht jedoch darin dass von dem Magneten eine starkes Hintergrundfeld ausgeht und der Sensor vor diesem Hintergrund messen muss. Dadurch wird es unmöglich, sehr kleine Veränderungen zu messen.

Die der Erfindung zugrunde liegende Aufgabe bestand darin, ein neues und einfaches Verfahren zum Nachweis von Analyten sowie dafür geeignete Reagenzien und Geräte bereitzustellen.

Bei der vorliegenden Erfindung wird das Vorhandensein bzw. die Konzentration eines nachzuweisenden Analyten mithilfe von magnetisierbaren und nicht-magnetisierbaren Beads bestimmt, wobei die Beads mit Bindemolekülen beschichtet sind, die abhängig vom Vorhandensein und/oder der Konzentration des Analyten in der Probe eine Aggregation bzw. Vernetzung eingehen können. Die Ausprägung dieser Aggregation bzw. Vernetzung wird magnetisch detektiert, womit ein in der Probe vorhandener Analyt qualitativ und/oder quantitativ bestimmt werden kann.

Ein erster Aspekt der Erfindung betrifft ein Verfahren zum Nachweis eines Analyten in einer Probe, umfassend die Schritte:

(a) Inkontaktbringen der Probe mit magnetisierbaren und nicht-magnetisierbaren Beads, wobei die Beads mit Bindemoleküle beschichtet sind, und wobei die magnetisierbaren Beads mit Molekülen vom Typ A beschichtet sind und die nicht-magnetisierbaren Beads mit Molekülen vom Typ B beschichtet sind, so dass die Analytmoleküle eine Verbindung zwischen den nicht-magnetisierbaren und den magnetisierbaren Beads herstellen und abhängig vom Vorhandensein und/oder der Konzentration des Analyten in der Probe eine Aggregation der magnetisierbaren Beads erfolgt, und

(b) Bestimmen des Aggregationsgrades der magnetisierbaren Beads durch magnetische Detektion, dadurch gekennzeichnet,

dass die Schritte (a) und (b) in einer Testkavität durchgeführt werden, wobei Schritt (b) das Anlegen eines Magnetfeldes umfasst, so dass sich die Testkavität in einem starken stationären. Magnetfeld befindet, welches hauptsächlich in horizontaler Richtung verläuft und dessen Betrag nach unten hin zunimmt, wodurch eine Bewegung der magnetisierbaren Beads hervorgerufen wird, so dass sich eine Schicht aus Beads bzw. Bead-Aggregaten ausbildet, die in Richtung der Feldlinien des externen Magnetfelds magnetisiert sind und ein zusätzliches Magnetfeld erzeugen, das unter Verwendung eines Magnetfeldsensors gemessen wird, der so unter der Testkavität positioniert ist, dass die vertikale Komponente des von der Beadschicht erzeugten Magnetfelds gemessen werden kann, wobei der Magnetfeldsensor nicht mittig unter der Testkavität, sondern in N-S-Richtung versetzt ist.

Weiterhin wird hierin ein Reagenz zum Nachweis eines Analyten in einer Probe beschrieben, welches eine oder mehrere Spezies von magnetisierbaren Beads umfasst, wobei die Beads mit Bindemolekülen beschichtet sind, so dass abhängig vom Vorhandensein und/oder der Konzentration des Analyten in der Probe eine Aggregation der magnetisierbaren Beads erfolgen kann.

Das Reagenz kann in einem Verfahren zur magnetischen Detektion eines Analyten, insbesondere in einem Verfahren wie zuvor beschrieben eingesetzt werden.

Ebenfalls beschrieben wird hierin eine Vorrichtung zum Nachweis eines Analyten in einer Probe, umfassend

(a) eine Testkavität,
(b) einen Magneten zum Erzeugen eines Magnetfeldes in der Testkavität und
(c) einen magnetischen Sensor zur Messung zeitlicher Veränderungen des Magnetfeldes in der Testkavität.

Die Vorrichtung kann in einem Verfahren zur magnetischen Detektion eines Analyten, insbesondere in einem Verfahren wie zuvor beschrieben eingesetzt werden.

Die Erfindung betrifft den Nachweis eines Analyten in einer Probe. Der Analyt kann prinzipiell eine beliebige Substanz sein, die durch Bindung an ein oder mehrere spezifische Bindepartner nachweisbar ist. Beispielsweise ist der Analyt ein Protein, z.B. ein Antikörper, ein Rezeptor, ein Rezeptorligand, ein Enzym etc., eine Nukleinsäure, z.B. eine DNA oder RNA, ein Hormon, ein Signaltransmitter, ein Metabolit, ein Medikament, eine Droge oder ein Pathogen wie etwa ein Virus oder Bakterium, oder jede andere Art von nachweisbarer Substanz. Vorzugsweise wird das Verfahren eingesetzt zum Nachweis von Antikörpern, beispielsweise Antikörpern gegen Pathogene, Autoimmunantigenen, Stoffwechselmetaboliten etc.

Die Probe ist vorzugsweise biologischen Ursprungs, z.B. eine Körperflüssigkeits-Probe wie etwa Blut, Serum, Plasma, Urin, Speichel, Liquor etc., eine Gewebeprobe, eine Zellkulturprobe, eine forensische Probe, eine Umweltprobe etc.

Gemäß der vorliegenden Erfindung werden magnetisierbare und nicht magnetisierbare Beads verwendet. Vorzugsweise handelt es sich bei den magnetisierbaren Beads um magnetisierbare Nanobeads. Die Beads haben vorzugsweise eine Größe im Bereich von 1-10.000 nm, besonders bevorzugt 5-1000 nm und am meisten bevorzugt 10-100 nm. Die Beads sind magnetisierbar. Vorzugsweise sind die Beads paramagnetische und/oder superparamagnetische Beads. Sie können z.B. aus Kobalt bestehen bzw. Kobalt enthalten. Andere mögliche Materialien sind Eisen bzw. Eisenoxid (z.B. Magnetit oder Eisenlegierungen). In einer bevorzugten Ausführungsform sind die Beads paramagnetische und/oder superparamagnetische Beads, die aus Magnetit bestehen bzw. Magnetit enthalten. Als besonders vorteilhaft haben sich Polymerbeads herausgestellt, die Magnetitpartikel enthalten. Vorteilhafterweise werden Beads mit einer hohen Sättigungsmagnetisierung, z.B. 1-200 emu/g, bevorzugt 2-70 emu/g oder 50-200 emu/g verwendet, um ein möglichst starkes nachzuweisendes Signal zu erzeugen.

Die Beads sind mit Bindemolekülen beschichtet, wobei die magnetisierbaren Beads mit Molekülen vom Typ A beschichtet sind und die nicht-magnetisierbaren Beads mit Molekülen vom Typ B beschichtet sind, so dass die Analytmoleküle eine Verbindung zwischen den nicht-magnetisierbaren und den magnetisierbaren Beads herstellen und abhängig vom Vorhandensein und/oder der Konzentration des Analyten eine Vernetzung bzw. Aggregation der magnetisierbaren Beads erfolgen kann.

In bestimmten Ausführungsformen werden Bindemoleküle verwendet, die spezifische Bindepartner des Analyten darstellen, d.h. eine spezifische Wechselwirkung mit dem Analyten eingehen, z.B. über eine Antikörper/ Antigen-Bindung, eine Rezeptor/Ligand-Bindung oder über eine Nukleinsäurehybridisierung. Wenn der Analyt beispielsweise ein Antikörper ist, können die zum Nachweis verwendeten Beads mit einem von dem Antikörper erkannten Antigen beschichtet sein. Wenn andererseits der Analyt ein Antigen ist, können die Beads mit einem gegen das Antigen gerichteten Antikörper oder einem Antikörperfragment beschichtet sein. Wenn der Analyt eine Nukleinsäure ist, können die Beads mit zum Analyten komplementären Nukleinsäuren (oder Nukleinsäureanaloga) beschichtet sein.

In anderen Ausführungsformen können Bindemoleküle verwendet werden, die indirekt, d.h. über einen nicht-immobilisierten Bindepartner, spezifisch mit dem Analyten binden. So können "universelle" Bindemoleküle, z.B. Antikörper gerichtet gegen die konstante Domäne von Antikörpern einer bestimmten Spezies (z.B. Maus, Ratte etc.), auf den Beads immobilisiert werden. Als Nachweisantikörper, die spezifisch mit dem Analyten bindefähig sind, werden dann nicht-immobilisierte Antikörpern dieser bestimmten Spezies verwendet.

In noch weiteren Ausführungsformen können die Beads auch mit den Analyten selbst oder einem Analogon des Analyten beschichtet sein, z.B. zur Verwendung in einem kompetitiven Test.

Für die vorliegende Erfindung kann eine einzige Spezies von magnetisierbaren Beads, d.h. eine einzige Sorte von Beads beschichtet mit einem Bindemolekül, oder mehrere Spezies von Beads, d.h. mehrere Sorten von Beads beschichtet mit unterschiedlichen Bindemolekülen, verwendet werden.

Weiterhin können dem Testansatz auch nicht-immobilisierte Bindemoleküle zugesetzt werden, z.B. nicht-immobilisierte Bindepartner für den Analyten, oder Verbindungsmoleküle zwischen Beads, die mit der Bindung des Analyten an Beads kompetieren.

Die Beschichtung von Beads mit Bindemolekülen kann durch bekannte Methoden erfolgen, z.B. durch Adsorption, durch kovalente Bindung, z.B. unter Verwendung chemischer Kopplungsreagenzien, und/oder durch hochaffine Wechselwirkungen, z.B. über eine Biotin/Streptavidin-Bindung.

In bestimmten Ausführungsformen kann es bevorzugt sein, die Beads mit einer Grundbeschichtung, vorzugsweise mit einer hydrophilen Grundbeschichtung zu versehen, um unspezifische Selbstaggregation aufgrund hydrophober und/oder magnetischer Wechselwirkungen zu verringern. In anderen Ausführungsformen kann der Effekt der unspezifischen Aggregation auch genutzt werden, um die Konzentration des Analyten zu bestimmen.

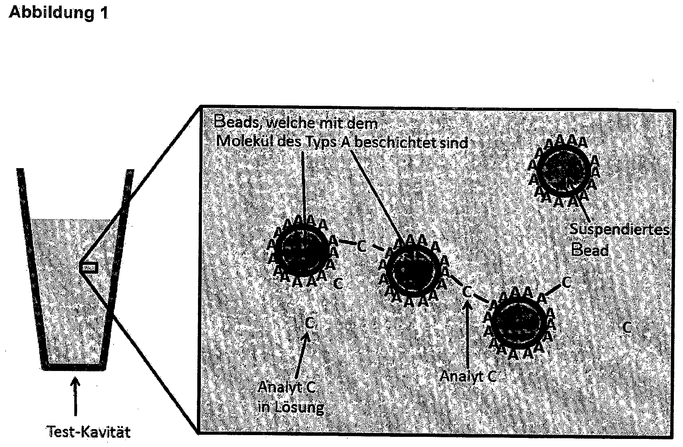

Im Folgenden werden verschiedene Varianten von Testformaten näher erläutert. Eine erste, nicht erfindungsgemäße Variante betrifft den Nachweis eines Analyten mit mehreren Bindungsstellen für ein Bindemolekül, beispielsweise den Nachweis eines Antikörpers. Diese Variante ist in **Abbildung 1** dargestellt. Besitzen Moleküle des Typs C (Analyt) mehrere Bindungsstellen für Moleküle des Typs A (Bindemolekül), so wird wie folgt verfahren: es werden Beads mit Molekülen des Typs A beschichtet. Vorzugsweise wird dann eine homogene Bead-Suspension hergestellt. Anschließend werden die Beads und eine Proben-Flüssigkeit, welche den Analyten C in einer nachzuweisenden Konzentration c enthält, in eine Testkavität eingebracht. Die Moleküle des Typs C binden an die Moleküle des Typs A auf der Oberfläche der Beads. Da der Analyt C mehrere Bindungsstellen für A enthält, findet eine Aggregation der Beads statt. Je höher die Konzentration c des Analyten C ist, desto größer ist der Aggregationsgrad der Beads.

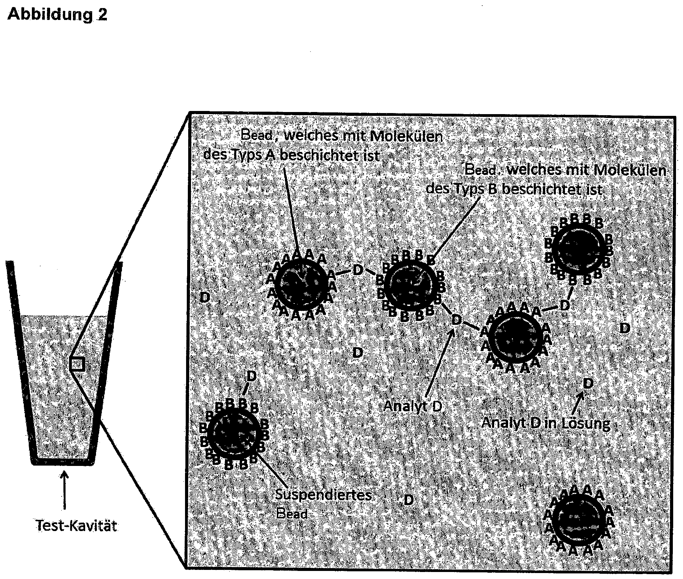

In einer weiteren Variante wird ein Testformat verwendet, welches die Bestimmung eines Analyten mit Bindungsstellen für zwei verschiedene Bindemoleküle ermöglicht. Diese Variante ist in **Abbildung 2** dargestellt. Besitzen Moleküle des Typs D (Analyt) eine oder mehrere Bindungsstellen für Bindemoleküle des Typs A und eine oder mehrere Bindungsstellen für Bindemoleküle des Typs B, so wird ein Teil der Beads mit Molekülen des Typs A (= "Beads Typ A") und ein anderer Teil der Beads mit Molekülen des Typs B (= "Beads Typ B") beschichtet. Vorzugsweise wird zunächst eine homogene Bead-Suspension mit Typ A- und Typ B-Beads hergestellt. Die Konzentrationen der beiden Bead-Typen sind zuvor hinsichtlich der Bindungsaffinitäten zwischen A und D sowie zwischen B und D optimiert worden. Anschließend werden die Beads und eine Proben-Flüssigkeit, die den Analyten D in einer nachzuweisenden Konzentration c enthält, in eine Testkavität eingebracht. Die Moleküle des Typs D binden an Moleküle des Typs A auf der Oberfläche der Beads des Typs A und an Moleküle des Typs B auf der Oberfläche der Beads des Typs B, wodurch eine Vernetzung bzw. Aggregation der Beads stattfindet. Der Aggregationsgrad ist umso größer, je größer die Konzentration c des Analyten D ist.

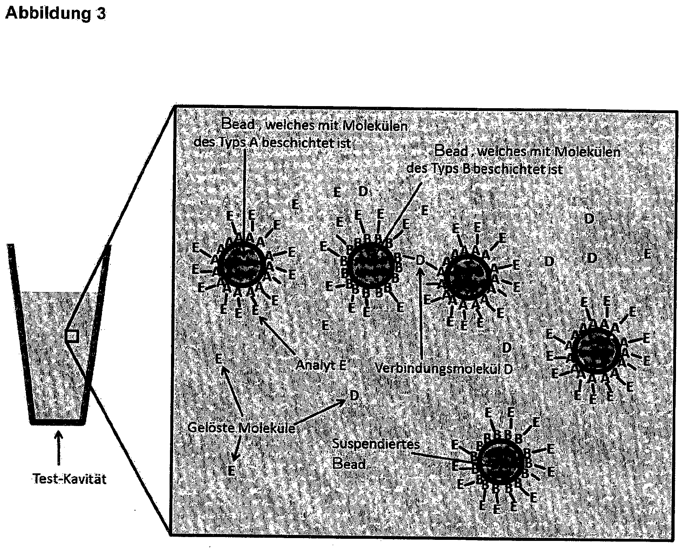

Noch eine weitere hier beschriebene Variante umfasst eine Hemmung der Aggregatbildung bei Vorhandensein des nachzuweisenden Analyten in der Probe. Diese Variante ist in **Abbildung 3** dargestellt. Moleküle des Typs E (Analyt) besitzen eine oder mehrere Bindungsstellen für Bindemoleküle des Typs A und eine oder mehrere Bindungsstellen für Bindemoleküle des Typs B, wobei im Fall von mehr als einer Bindungsstelle eine Bindung von E an A die Ausbildung einer Bindung von E an B hemmt und die Ausbildung einer Bindung zwischen E an B die Ausbildung einer Bindung von E an A hemmt. Ein Teil der in dieser Variante verwendeten Beads ist mit Molekülen des Typs A beschichtet und ein anderer Teil der Beads ist mit Molekülen des Typs B beschichtet. Vorzugsweise wird zunächst eine homogene Bead-Suspension mit Typ A- und Typ B-Beads hergestellt, wobei die Konzentrationen der beiden Bead-Typen vorteilhafterweise zuvor wie oben beschrieben optimiert werden. Anschließend werden die Beads und eine Proben-Flüssigkeit, die den Analyten E in einer nachzuweisenden Konzentration c enthält, in eine Testkavität eingebracht. In Abwesenheit des Verbindungsmoleküls D findet keine Vernetzung bzw. Aggregation der Beads statt. Erst in Anwesenheit von Verbindungsmolekülen des Typs D kann eine Aggregation stattfinden. Durch Bindung des Analyten E an die auf den Beads immobilisierten Bindemoleküle des Typs A oder B wird die Aggregation von Beads gehemmt, weil weniger Bindungsstellen für Verbindungsmoleküle des Typs D frei sind. Je höher die Konzentration von E ist, desto niedriger ist der Aggregationsgrad der Beads.

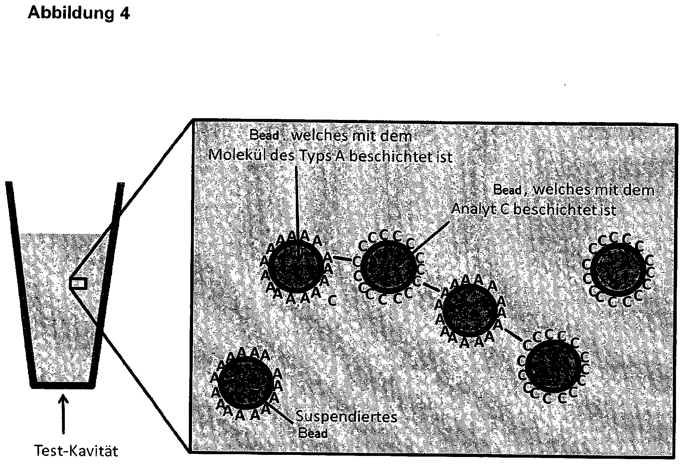

In noch einer weiteren Variante kann der Analyt oder ein Analytanalogon auf dem Bead selbst immobilisiert sein. Diese Ausführungsform ist in **Abbildung 4** gezeigt. In diesem Fall gibt es Beads, welche mit dem Bindemolekül A beschichtet sind und Beads, welche mit dem Analyten C oder einem Analogon davon beschichtet sind. Vorzugsweise werden zunächst homogene Bead-Suspensionen mit Typ A- und Typ C-Beads hergestellt, wobei die Konzentrationen der beiden Bead-Suspensionen vorteilhafterweise zuvor optimiert worden sind. Anschließend werden vorzugsweise die Beads des Typs C und eine Proben-Flüssigkeit, die den Analyten C in einer nachzuweisenden Konzentration c enthält, in eine Testkavität eingebracht. Nach Zugabe von Beads des Typs A kann eine Aggregation stattfinden, wobei der Aggregationsgrad mit zunehmender Konzentration des Analyten C abnimmt.

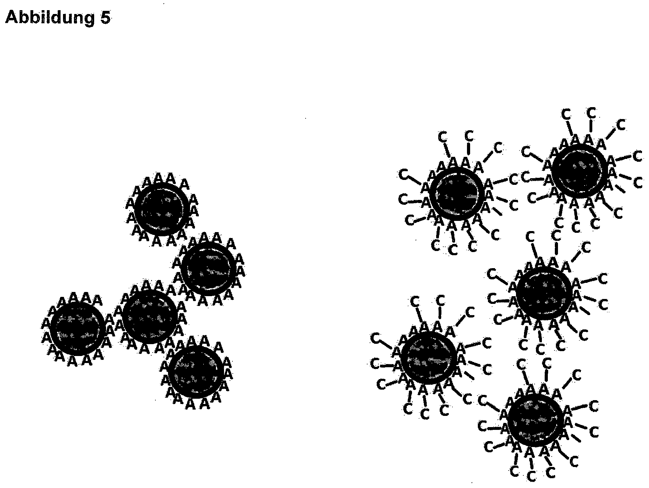

In noch einer weiteren Variante kann der Effekt der unspezifischen Selbstaggregation zur Bestimmung der Konzentration des Analyten verwendet werden. Diese Variante ist in **Abbildung 5** gezeigt. Hier liegen Beads vor, die mit Bindemolekülen des Typs A beschichtet sind. Es besteht eine Tendenz zur Selbstaggregation (links). Bei Anlagerung von Analytmolekülen C, die eine einzige Bindungsstelle für das Bindemolekül A besitzen, wird die Selbstaggregation gehemmt, da der Abstand zwischen den einzelnen Beads größer ist. Bei zunehmender Konzentration des Analyten C nimmt der Aggregationsgrad ab (rechts).

Erfindungsgemäß liegen zwei verschiedene Bead-Typen in Suspension vor: zum Einen magnetisierbare Beads, welche mit Molekülen des Typs A beschichtet sind, zum Anderen nicht-magnetisierbare Beads, welche mit Mokeülen des Typs B beschichtet sind. Die nicht-magnetisierbaren Beads können z.B. aus Polymer (z.B. Polystyrol), Stärke, Silan, etc., bevorzugt aus Polymer, insbesondere aus Polystyrol, bestehen.

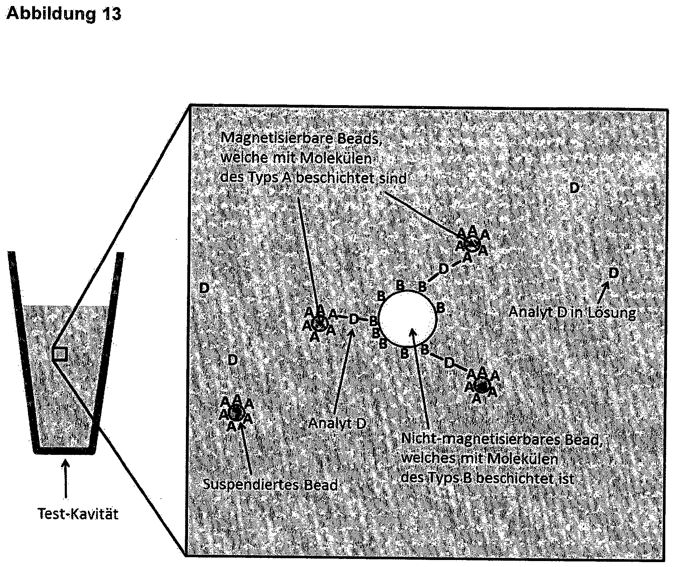

Nachzuweisende Analyt-Moleküle des Typs D stellen eine Verbindung zwischen nicht-magnetisierbaren und magnetisierbaren Beads her. Je höher die Konzentration c des Analyten D ist, desto mehr magnetisierbare Beads binden an nicht-magnetisierbare Beads. Das Prinzip ist in **Abbildung 13** veranschaulicht.

Bei der erfindungsgemäßen Vorgehensweise mit zwei verschiedenen, magnetisierbaren sowie nicht-magnetisierbaren, Bead-Typen wirkt sich die Konzentration des Analyten auf die Geschwindigkeit aus, mit welcher sich der Bead-Layer ausbildet. Sind die magnetisierbaren Beads an nicht-magnetisierbare Beads gebunden, so bewegen sie sich im magnetischen Feld langsamer.

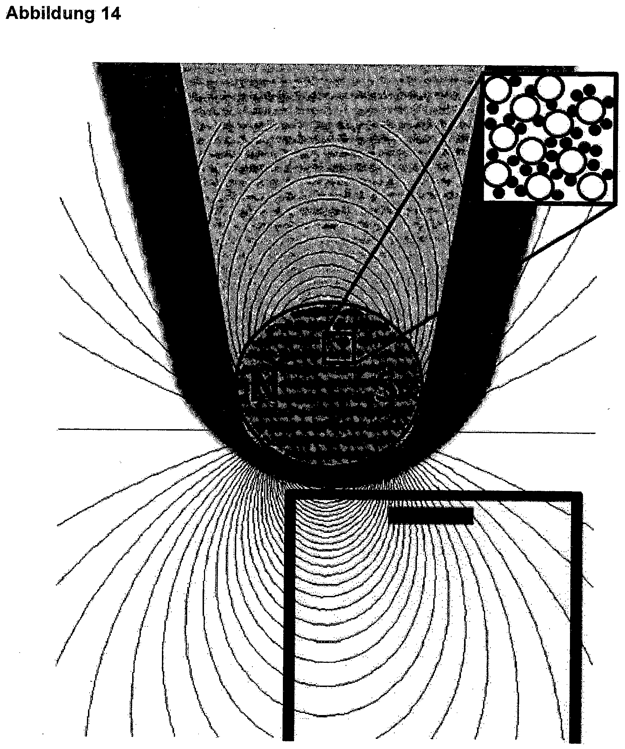

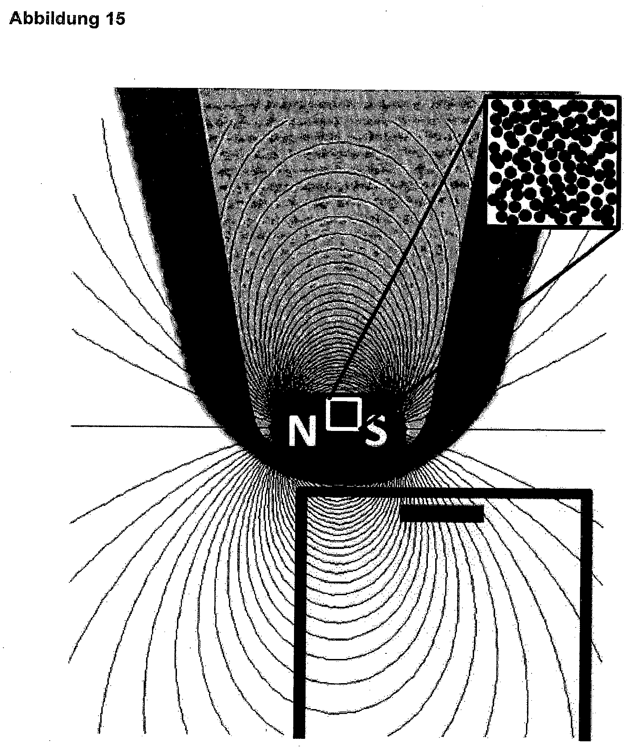

In der Ausführungsform mit zwei verschiedenen magnetisierbaren sowie nicht-magnetisierbaren Bead-Typen wirkt sich die Analyt-Konzentration auch auf die absolute Magnetfeldstärke aus. Gibt es viele Bindungen von magnetisierbaren an nicht-magnetisierbare Beads, so ist die Dichte der magnetisierbaren Beads im Bead-Layer geringer, wodurch das magnetische Signal kleiner ist (siehe **Abbildung 14).** Gibt es keine solche Bindungen, so ist die Dichte der magnetisierbaren Beads im Bead-Layer höher, wodurch das magnetische Signal größer ist (siehe **Abbildung 15).** Entsprechende Abstufungen sind möglich. Insgesamt gilt: je mehr Analyt-Moleküle in der Bead-Suspension vorhanden sind, desto geringer ist die Dichte der magnetischen Beads im Layer und desto kleiner ist das magnetische Signal.

Die Ausführungsform mit zwei verschiedenen magnetisierbaren sowie nicht-magnetisierbaren Bead-Typen eignet sich auch zur Trennung von gebundenen und ungebundenen Beads. Die nicht-magnetisierbaren Beads können aus einem Material von geringer Dichte knapp über der Dichte von Wasser (p: 1,01-2,59 g/cm$^3$) bestehen. Wird nun die Flüssigkeitsdichte über die Dichte der nicht-magnetisierbaren Beads erhöht (z.B. mittels Zugabe von Sucrose, Poly-Sucrose (z.B. Ficoll), Glycerol, verschiedene Polyole, etc.), so schwimmen die Beads auf. Ist der Auftrieb groß genug, so schwimmen die nicht-magnetisierbaren Beads auch dann noch auf, wenn magnetisierbare Beads angebunden haben. Vor Einsetzen der Mess-Kavität in das Magnetfeld kann gewartet werden, bis die Beads aufgeschwommen sind. Sie befinden sich dann so weit oben, dass sie durch das Magnetfeld nicht nach unten gezogen werden, da dieses hier zu schwach ist. Auch ohne Warten befindet sich ein Teil der Beads außerhalb der Reichweite des Magnetfeldes. Auf diese Weise können ungebundene von gebundenen Beads separiert werden. Je höher die Analyt-Konzentration ist, desto kleiner ist das magnetische Signal.

Statt nicht-magnetisierbarer Beads können auch Polymer-Moleküle eingesetzt werden, welche mit Molekülen des Typs B funktionalisiert sind. Diese stellen dann eine Aggregation zwischen den magnetisierbaren Beads her, sofern Analyt-Moleküle vorhanden sind. Eine Bestimmung der Analyt-Konzentration ist wiederum mittels einer kinetischen Messung möglich.

Bei dem Polymer-Molekül kann es sich z.B. um ein synthetisches oder natürliches Polymer, beispielsweise um Stärke(-Derivate), Dextran(-Derivate), Polyethylenglycol, Polyacrylamid etc. handeln.

Die verschiedenen Ausführungsformen des erfindungsgemäßen Nachweissystems zeichnen sich dadurch aus, dass sich der Aggregationsgrad der Beads abhängig vom Vorhandensein und/oder der Konzentration des nachzuweisenden Analyten in der Probe ändert.

Der Nachweis wird in einer Testkavität durchgeführt, in welche die Probenflüssigkeit und die magnetisierbaren und nicht-magnetisierbaren Beads, vorzugsweise als Suspension, eingebracht worden sind. Darüber hinaus können gegebenenfalls Puffer und/oder weitere Reagenzien vorhanden sein. Die Testkavität hat vorzugsweise ein Volumen von 1-1.000 µl, besonders bevorzugt von 50-300 µl. Sie kann - je nach Testformat - unterschiedlich ausgeformt sein, bevorzugt hat sie eine langgestreckte Form, z.B. mit einer Länge von 3 mm bis 50 mm, um die Geschwindigkeitsunterschiede zwischen Nanobeads und Nanobead-Aggregaten gut auflösen zu können und um Flüssigkeitsvolumen zu sparen.

Schritt (b) des erfindungsgemäßen Verfahren umfasst das Bestimmen des Aggregationsgrades der magnetisierbaren Beads durch magnetische Detektion. Dabei umfasst Schritt (b) das Erzeugen eines Magnetfeldes in der Testkavität, das eine Bewegung der magnetisierbaren Beads hervorruft. Besonders bevorzugt umfasst die Detektion des Aggregationsgrades der Beads eine Bestimmung der Bewegung der magnetisierbaren Beads in einem inhomogenen Magnetfeld.

Der Nachweis des Analyten in der Probe beruht darauf, dass sich vernetzte Bead-Aggregate in einem Magnetfeld mit einer anderen Geschwindigkeit als einzelne Beads bewegen. Vorzugsweise erfolgt die Bewegung dabei in einem Magnetfeld-Gradienten in Richtung zunehmender Magnetfeldstärke. Auch große vernetzte Bead-Aggregate bewegen sich mit einer anderen Geschwindigkeit, z.B. in Richtung des stärker werdenden Magnetfeldes, als kleine vernetzte Bead-Aggregate.

Daher wandern Beads bzw. Bead-Aggregate aus der Suspension zu den Stellen der Messkavität und sammeln sich dort, an denen das angelegte Magnetfeld $\vec{B_1}(\vec{r})$ am stärksten ist, und zwar mit einer Geschwindigkeit, die davon abhängt, wie groß der Vernetzungsgrad zwischen den Beads ist.

Die Kraft, welche auf die Beads wirkt, resultiert vorzugsweise aus einem Feldgradienten im Magnetfeld. Der Feldgradient kann durch ein inhomogenes Magnetfeld erzeugt werden, in dem die Beads dann in Richtung des stärker werdenden Magnetfeldes beschleunigt werden.

Da die Beads in dem externen Magnetfeld $\vec{B_1}(\vec{r})$ magnetisiert werden, erzeugen sie ihrerseits ein Magnetfeld, welches zu einem zeitabhängigen Zusatzfeld $\Delta\vec{B}(c, t, \vec{r})$ führt, welches das externe Feld $\vec{B_1}(\vec{r})$ überlagert. Diese zeitabhängige Veränderung wird gemessen ("kinetische Messung"). Die zeitliche Entwicklung des Zusatzfeldes ist abhängig vom Aggregations- bzw. Vernetzungsgrad der Beads und somit von der Konzentration des Analyten.

Vorteilhafterweise werden Beads mit einer möglichst hohen Sättigungsmagnetisierung verwendet, um ein möglichst großes Zusatzfeld und dementsprechend ein starkes nachzuweisendes Signal zu erzeugen.

Vorzugsweise umfasst die Analytbestimmung durch das erfindungsgemäße Verfahren eine Kalibrierung des Systems, wobei der Aggregations- bzw. Vernetzungsgrad der Beads in Kontrollproben ohne Analyten bzw. mit einer oder mehreren bekannten Analytkonzentrationen bestimmt wird. Zur Kalibrierung des Systems werden vorzugsweise Mischungen aus Bead-Suspension und Probenflüssigkeiten, welche den Analyten in definierten Konzentrationen $c_i$ enthalten, hergestellt. Mit diesen Mischungen werden kinetische Kurven $\Delta(\vec{B})(c_i, t, \vec{r_0})$ bzw. $|\Delta(\vec{B})(c_i, t, \vec{r_0})|$ aufgezeichnet, wobei $\vec{r_0}$ die Position des Magnetfeld-Sensors ist. Die den Verlauf der kinetischen Kurven definierenden Parameter werden gemessen bzw. durch einen Kurvenfit bestimmt.

In vielen Fällen lässt sich ein Teil der kinetischen Kurve durch eine Funktion der Form

$$\left|\vec{B}_{Fit}(t)\right| = P + Q \cdot \left(1 - e^{-\frac{t}{\tau(c)}}\right) \qquad (1)$$

beschreiben. Die Zeitkonstante T(c) ist konzentrationsabhängig und die Kurve T(c) wird experimentell bestimmt (Aufnahme der sog. "Standardkurve"). Die Standardkurve wird durch einen weiteren Kurvenfit an die diskreten Messwerte $T_i$(c) erstellt.

Eine weitere wichtige Kinetik folgt der Langmuir-Gleichung

$$\left|\vec{B}_{Fit}(t)\right| = P \cdot \frac{t/\tau(c)}{1 + t/\tau(c)} \qquad (2)$$

Es sind auch andere kinetische Verläufe denkbar, die z.B. mit einer polynomialen Funktion angefittet werden können.

Liegt nun Probenflüssigkeit mit einer unbekannten Konzentration $c_\times$ des Analyten vor, so wird zunächst eine kinetische Kurve aufgenommen, welche mit einem Kurvenfit gemäß Gleichung (1) oder gegebenenfalls Gleichung (2) gefittet wird. Mittels der auf diese Weise bestimmten Zeitkonstante wird über die Zuordnungsfunktion c→T(c) bzw. T→c(T) die Konzentration $c_\times$ berechnet.

In bestimmten Ausführungsformen des Verfahrens kann die Detektion des Aggregationsgrades die Verwendung einer Testkavität mit einem Filterelement, z.B. einem Sieb, beinhalten. Das Filterelement weist dabei eine größere Durchlässigkeit für nicht aggregierte Beads als für Bead-Aggregate auf. Der Durchmesser der Löcher des Filterelements kann beispielsweise im Bereich von etwa 100 nm bis etwa 10 $\mu$m liegen.

Bei Verwendung eines Filterelements können Unterschiede in den Geschwindigkeiten einzelner Beads, kleinerer Bead-Aggregate und größerer Bead-Aggregate zusätzlich vergrößert werden. Ab einer bestimmten Größe werden die Aggregate vom Filterelement komplett zurückgehalten, so dass dann auch das absolute magnetische Signal S in der Sättigung der Kinetik reduziert wird. Damit kann auch S als ein Maß für die Konzentration eines Analyten verwendet werden. S entspricht $\left|\vec{B}_{Fit}\right|$ nach einer genügend langen Zeit t. Analog zum vorhergehenden Abschnitt kann so eine Standardkurve S(c) erstellt werden. Das Vorhandensein und die Bestimmung der Konzentration eines Analyten kann wie zuvor beschrieben erfolgen.

Damit die Löcher des Filterelements nicht in zu großem Maße von Bead-Aggregaten verstopft werden, kann die Testkavität mechanisch oder mittels Ultraschall-Anregung bewegt werden.

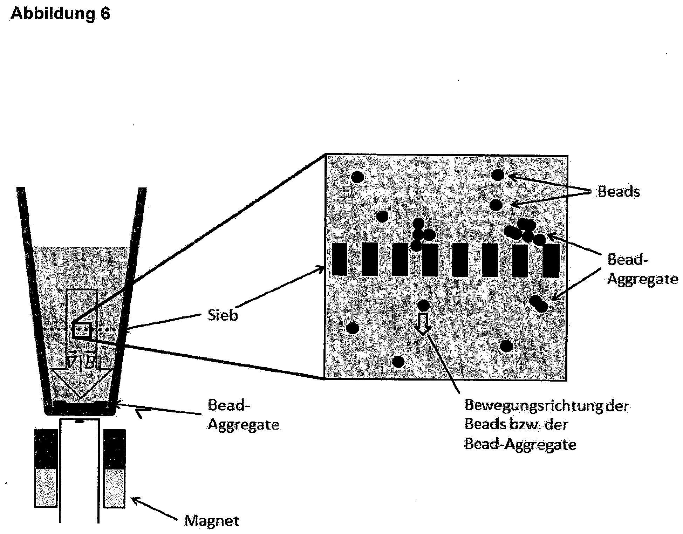

**Abbildung 6** zeigt eine Testkavität mit einem eingebauten Sieb. Durch einen Magneten wird in der Testkavität ein Magnetfeld erzeugt. Im linken Abschnitt der Abbildung ist die zunehmende Feldstärke durch den Pfeil $\vec{\nabla}\left|\vec{B}\right|$ gekennzeichnet. Die Beads wandern wie zuvor beschrieben in Richtung zunehmender Magnetfeldstärke. Dabei können einzelne Beads im Wesentlichen ungehindert durch das Sieb hindurch treten, während Bead-Aggregate langsamer durch das Sieb wandern bzw. ab einer gewissen Größe überhaupt nicht durch das Sieb hindurchtreten können. Im rechten Abschnitt von Abbildung 6 ist die Bewegungsrichtung exemplarisch für ein Bead angegeben.

Zur Messung des Magnetfeldes bzw. von Änderungen des Magnetfeldes wird ein Magnetfeldsensor verwendet. Geeignete Sensoren sind z.B. Hall-Sensoren oder magnetoresistive Sensoren.

Statt eines Magnetfeld-Sensors kann auch ein anderer Sensor verwendet werden, um den Bead-Layer zu detektieren. Mögliche Messgrößen sind z.B. die Impedanz und die Induktivität einer Spule bzw. einer Spulenanordnung unter dem Bead-Layer.

Prinzipiell ist es vorteilhaft, den Magnetfeld-Sensor so nah wie möglich an den Bead-Aggregaten zu positionieren und die Beads möglichst stark zu magnetisieren.

Es gibt Magnetfeld-Sensoren, mit denen extrem kleine Felder gemessen werden können. Die Feldbeeinflussung durch die Bead-Aggregate liegt im Abstand von bis zu wenigen Millimetern im Nano- und Mikrotesla-Bereich.

Um die vom Aggregationsgrad der Beads abhängige Magnetfeldveränderung zu messen, kann die Verwendung eines ringförmigen Magneten bevorzugt sein, insbesondere eines Ringmagneten mit Nullfeld-Punkten. Der Ringmagnet mit Nullfeld-Punkten kann entweder ein Permanentmagnet oder ein Elektromagnet sein.

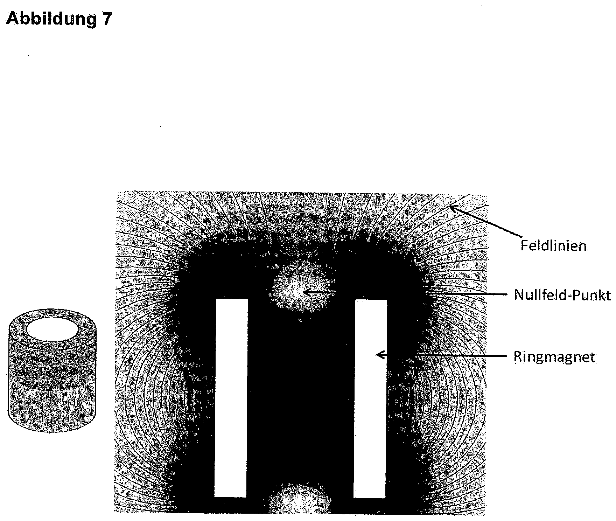

**Abbildung 7** zeigt die Feldverteilung in der Umgebung eines Ringmagneten. Der Ringmagnet ist im großen Bild im Querschnitt dargestellt. Je dunkler das Grau ist, desto stärker ist der Betrag des Magnetfeldes.

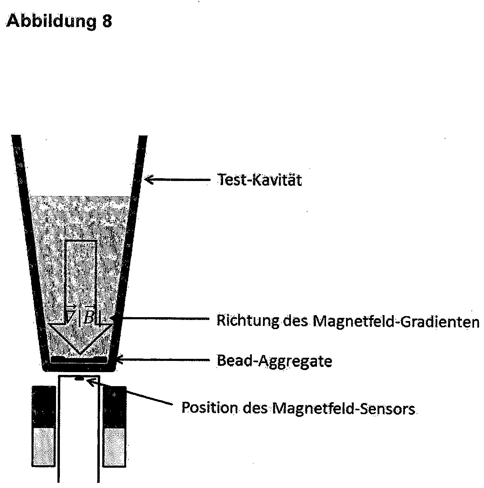

**Abbildung 8** zeigt den Aufbau einer Messanordnung mit Ringmagneten. Dabei wird in einer Testkavität ein Magnetfeld erzeugt, wobei die Richtung des Magnetfeldgradienten durch den Pfeil $\vec{\nabla}\left|\vec{B}\right|$ angegeben ist. Die Position des aktiven Bereichs der Magnetfeldsonde fällt mit dem oberen Nullfeld-Punkt zusammen. Die Beads bzw. Bead-Aggregate wandern in der Testkavität in Richtung des Magnetfeldgradienten, d.h. in Pfeilrichtung. Aufgrund der Ringform des Magneten sammeln sich die Beads vorzugsweise ringförmig am Boden der Testkavität. Der Grund dafür ist, dass das Magnetfeld hier stärker ist als in der Mitte. Vorteilhafterweise kann deswegen eine Testkavität mit zumindest teilweise flachem Boden verwendet werden. Erst wenn die Kavität einen gewissen Abstand zum Magneten hat, verschwindet dieser Effekt und es bildet sich eine gleichmäßige BeadSchicht am Boden (siehe Verteilung der Feldstärke in Abbildung 7).

In einer weiteren bevorzugten Ausführungsform kann das Magnetfeld durch einen Elektromagneten erzeugt werden, der mit zeitlichen Unterbrechungen arbeitet. Der Elektromagnet besteht im einfachsten Fall aus einer zylinderförmigen Spule, an welche je nach benötigter Feldstärke Metall-Komponenten angebracht werden können, um die Feldstärke zu erhöhen.

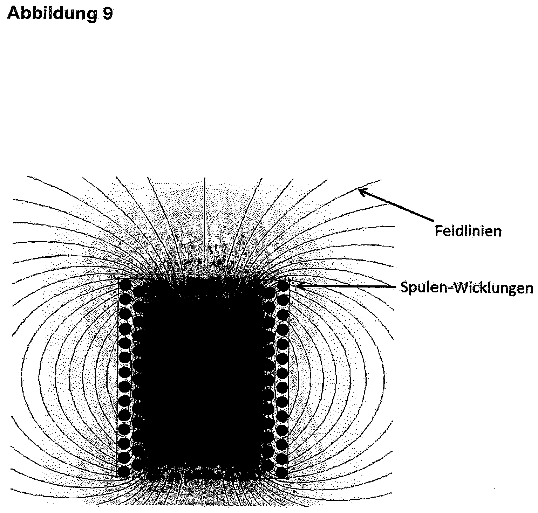

**Abbildung 9** zeigt die Feldverteilung in der Umgebung eines als Spule ausgebildeten Elektromagneten. Die Spule kann - gegebenenfalls zusammen mit feldverstärkenden Metall-Komponenten - unterhalb der Testkavität positioniert werden.

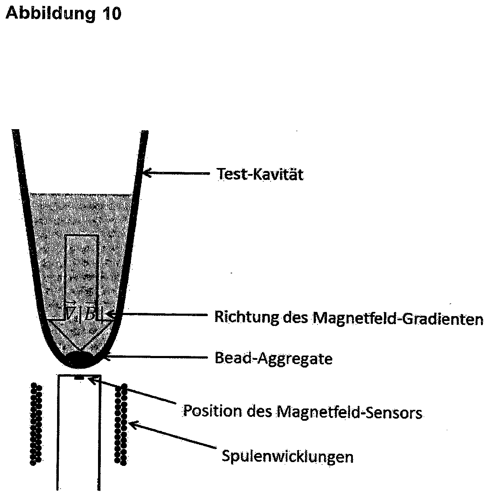

**Abbildung 10** zeigt den Aufbau einer solchen Messanordnung mit Elektromagnet. In der Testkavität wird durch den Elektromagneten ein Magnetfeld erzeugt. Die Richtung des Magnetfeld-Gradienten ist durch den Pfeil $\vec{\nabla}|\vec{B}|$ angegeben. Die Beads bzw. Bead-Aggregate wandern in der Testkavität in Richtung des Magnetfeld-Gradienten und sammeln sich am Boden der Testkavität. Im Vergleich zu der Variante mit Ringmagnet (siehe Abbildung 7) sammeln sich in diesem Fall mehr Beads auch in der Mitte des Bodens. Daher kann die Verwendung eines nach unten hin konisch zulaufenden Gefäßes vorteilhaft sein.

Um mit hoher Sensitivität messen zu können, kann der Elektromagnet in definierten Intervallen $\Delta t$ für einen definierten Zeitraum $\delta t$ deaktiviert werden. Mit dem Sensor wird dann die remanente Magnetisierung der Bead-Aggregate gemessen. Analog zur Ringmagnet-Version wird auf diese Weise eine kinetische Kurve aufgenommen, mit der auf die Geschwindigkeit der Ausbildung der Bead-Aggregate geschlossen werden kann.

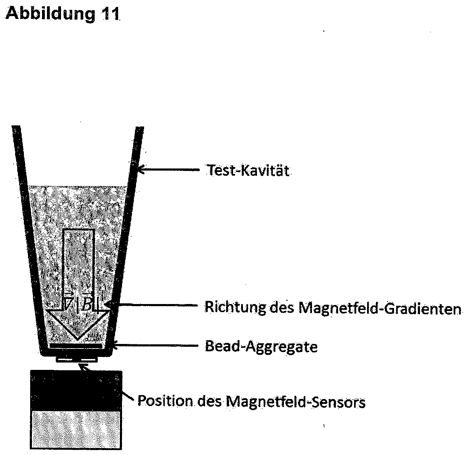

Es sind auch andere Ausführungen mit Permanent- oder Elektromagneten denkbar, welche andere Feld-Geometrien erzeugen. So kann es in bestimmten Fällen möglich sein, einen Permanent-Magneten oder einen Elektromagneten zu benutzen, welcher keine Nullfeld-Punkte besitzt, und diesen während der Messung nicht abzuschalten (siehe **Abbildung 11**). Denkbar ist z.B. die Verwendung eines sehr kleinen Gesamtfeldes $\vec{B}_1(\vec{r})$.

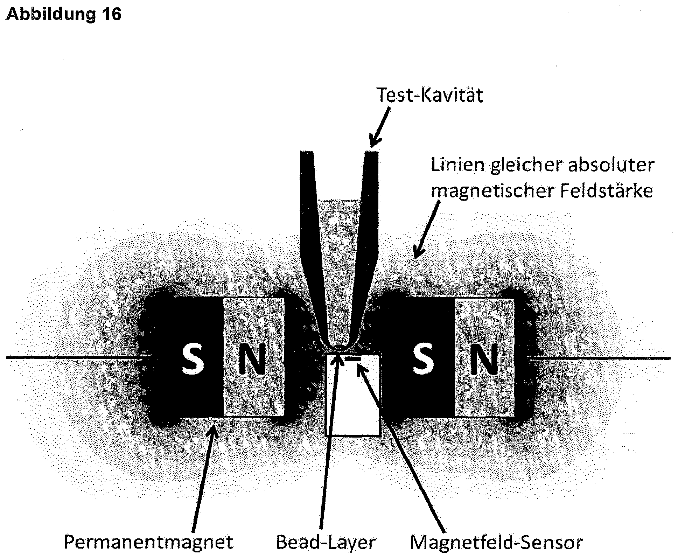

**Abbildung 16** zeigt die erfindungsgemäße Vorgehensweise, in der sich die Testkavität im einem starken stationären Magnetfeld, welches hauptsächlich in horizontaler Richtung verläuft und dessen Betrag nach unten hin zunimmt, befindet. Ein solches Feld kann durch zwei Permanent-Magnete erzeugt werden, die zu beiden Seiten der Testkavität positioniert sind (Abbildung 16).

In dieser Magnetfeld-Geometrie werden die magnetisierbaren Beads wiederum nach unten hin beschleunigt, so dass sich eine Schicht aus Beads bzw. Bead-Aggregaten ausbildet. Die Beads sind in Richtung der Feldlinien des externen Magnetfeldes magnetisiert und erzeugen daher ein zusätzliches Magnetfeld.

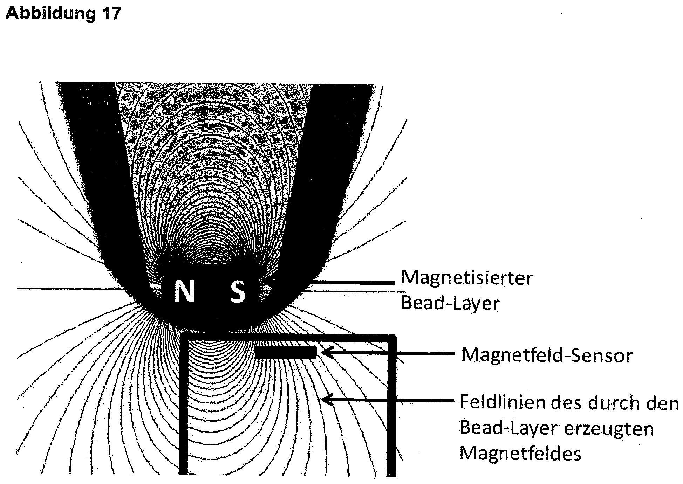

Der Magnetfeld-Sensor ist so unter der Test-Kavität positioniert, dass die vertikale Komponente des vom Bead-Layer erzeugten Magnetfeldes gemessen werden kann. Er befindet sich zu diesem Zweck nicht mittig unter der Test-Kavität, sondern in N-S-Richtung versetzt (siehe **Abbildung 17**). Die Test-Kavität hingegen befindet sich mittig zwischen den beiden Permanent-Magneten, damit sich die Beads möglichst unten sammeln.

Das Sammeln und Messen der Bead-Aggregate erfolgt vorzugsweise am Boden der Messkavität, ist aber auch an einer Seite der Messkavität möglich.

Gegebenenfalls können die Testkavitäten mittels Ultraschall angeregt werden, um die Geschwindigkeit, mit welcher die Bead-Aggregate sich sammeln, zu beeinflussen.

Die vorstehend beschriebenen Varianten, bei denen zwei verschiedene Bead-Typen in Suspension vorliegen, nämlich magnetisierbare Beads, welche mit Molekülen des Typs A beschichtet sind, und nicht-magnetisierbare Beads, welche mit Molekülen des Typs B beschichtet sind, können dafür verwendet werden, Analyt-gebundene Beads von ungebundenen Beads zu trennen. Es wird daher weiterhin auch ein Verfahren zur Abtrennung von Analyt beschrieben, umfassend die Schritte (i) Bereitstellen einer Suspension, umfassend magnetisierbare Beads, welche mit einem ersten Typ von Bindemolekülen beschichtet sind, nicht-magnetisierbare Beads, welche mit einem zweiten Typ von Bindemolekülen beschichtet sind, und mindestens ein Suspensionsmedium, wobei die Dichte der nicht-magnetisierbaren Beads $\rho_{nmB}$ im Bereich von 1,01-2,5 g/cm$^3$, bevorzugt 1,1-2,5 g/cm$^3$ liegt, (ii) Zugabe von Analyt zu der Suspension, (iii) Einstellen der Dichte des Suspensionsmediums auf einen Wert $\rho_{Medium} > \rho_{nmB}$, (iv) Abtrennen der Analyt-gebundenen Beads. Bevorzugt ist das Suspensionsmedium Wasser.

Durch Anpassen der Dichte des Suspensionsmediums über die Dichte der nicht-magnetisierbaren Beads, z.B. mittels Zugabe von Sucrose, Poly-Sucrose, z.B. Ficoll, Glycerol, Polyol, etc., schwimmen die Beads auf. Ist der Auftrieb groß genug, so schwimmen die nicht-magnetisierbaren Beads auch dann noch auf, wenn magnetisierbare Beads angebunden haben. Eine einfache Abtrennung der Analyt-gebundenen Beads von ungebundenen Beads kann somit erreicht werden. Dies kann auch verwendet werden, um die Analytkonzentration zu bestimmen. Hierzu wird die separierte Probe in die Mess-Kavität in das Magnetfeld eingesetzt. Die Analyt-gebundenen Beads befinden sich an der Oberfläche und können durch das Magnetfeld nicht nach unten gezogen werden, da dieses zu schwach ist. Je höher die Analytkonzentration ist, desto kleiner ist das magnetische Signal.

Zur Durchführung des erfindungsgemäßen Verfahrens eignet sich ein Reagenz, das eine oder mehrere Spezies von magnetisierbaren Beads enthält. Die Beads sind wie zuvor beschrieben mit Bindemolekülen beschichtet, so dass abhängig vom Vorhandensein und/oder der Konzentration des Analyten in der Probe eine durch magnetische Detektion nachweisbare Aggregation der magnetisierbaren Beads erfolgen kann. Darüber hinaus kann das Reagenz noch weitere Komponenten, z.B. Bindemoleküle oder Vernetzungsmoleküle in freier Form, sowie Zusatzstoffe, z.B. Puffer, Entstörreagenzien etc., enthalten.

Das Reagenz kann als weitere Bestandteile noch Kontrollproben zur Kalibrierung des Nachweissystems enthalten wie zuvor beschrieben.

Eine zur Durchführung des Verfahrens geeignete Vorrichtung umfasst eine Testkavität, einen Magneten zum Erzeugen eines Magnetfeldes, insbesondere eines inhomogenen Magnetfeldes bzw. eines Magnetfeld-Gradienten, in der Testkavität und einen magnetischen Sensor, der zur Messung zeitlicher Änderungen des Magnetfeldes in der Testkavität geeignet ist.

Des Weiteren kann die Vorrichtung eine Einheit zur Auswertung der durch den Sensor gemessenen Signale, z.B. einen Prozessor, sowie gegebenenfalls ein Gehäuse, eine Konsole etc. enthalten.

Die nachfolgenden Beispiele dienen zur Erläuterung der vorliegenden Erfindung.

**Beispiel 1 (Vergleichsbeispiel)**

Es wurden funktionalisierte Beads der Firma TurboBeads (www.turbobeads.com) verwendet. Die Beads bestehen aus Kobalt und haben eine Sättigungsmagnetisierung von etwa 158 emu/g. Der Durchmesser liegt bei etwa 30 nm. Die Kobalt-Beads sind mit einer Kohlenstoff-Schicht überzogen.

Für diesen Versuch wurden Beads verwendet, bei denen die Kohlenstoff-Schicht mit Biotin-Molekülen funktionalisiert ist. Als Test-Analyt wurde Streptavidin verwendet. Streptavidin besitzt vier Bindungsstellen für Biotin. Das so realisierte Testsystem entspricht der ersten beschriebenen Ausführungsform (siehe Abbildung 1)

Zur Messung der magnetischen Feldstärke diente das Teslameter FH 54 mit der Hallsonde HS-AGE5-4805 von der Firma MAGNET-PHYSIK (www.magnet-physik.de).

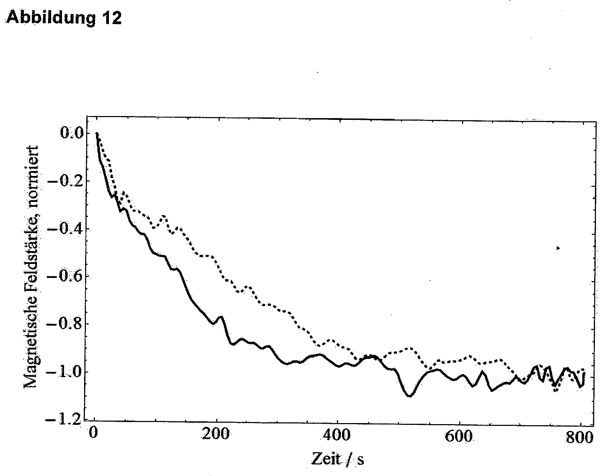

Als Versuchsaufbau wurde die Konfiguration aus Abbildung 8 verwendet, allerdings mit einer nach unten hin spitz zulaufenden Test-Kavität. **Abbildung 12** zeigt die zeitabhängige Veränderung der magnetischen Feldstärke am Ort des Magnetfeldsensors.

Zu Beginn der Messung befanden sich 500 $\mu$l homogene Nanobead-Suspension in der Mess-Kavität. Die Konzentration der Nanobeads betrug 2,67 $\cdot 10^{11}$ Nanobeads/ml, die Bindungskapazität lag bei 0,1 mmol/g.

Da sich ein Permanentmagnet unter der Test-Kavität befand, begannen die Beads unmittelbar nach Zugabe der Suspension, nach unten zu wandern. Die gemessene magnetische Feldstärke ist in Abbildung 12 als durchgezogene schwarze Linie dargestellt.

In einem weiteren Experiment wurde genauso verfahren mit dem einzigen Unterschied, dass die Bead-Suspension vor Zugabe mit einer Streptavidin-Lösung vermischt wurde. Streptavidin geht eine Bindung mit Biotin ein, so dass sich auf der Oberfläche der Beads eine Streptavidin-Schicht bildet. Da Streptavidin vier Biotin-Bindungsstellen besitzt, aggregieren die Nanobeads (siehe Abbildung 1). Die Streptavidin-Konzentration in der Bead-Suspension lag bei 333 pmol/ml.

Die aufgenommene magnetische Feldstärke ist als gestrichelte Kurve in Abbildung 12 dargestellt.

Es ist deutlich zu erkennen, dass die Nanobeads bzw. Nanobead-Aggregate im Experiment mit Streptavidin gegenüber dem Experiment ohne Streptavidin eine verlangsamte Kinetik aufweisen.

Dieser Effekt war reproduzierbar.

**Beispiel 2 (Vergleichsbeispiel)**

**Nachweis eines Analyten mit mehreren Bindungsstellen an Bindemoleküle (siehe Abbildung 1)**

Es wurde ein Aufbau verwendet, bei dem die Beads in ein horizontal verlaufendes Feld hineingezogen werden (siehe **Abbildung 16**).

Zur Messung der magnetischen Feldstärke diente das Teslameter FH 54 mit der Hallsonde HS-AGE5-4805 von der Firma MAGNET-PHYSIK (www.magnet-physik.de).

Es wurden magnetisierbare Beads der Firma Merck Millipore (Marke Estapor, www.estapor.com) mit einem Durchmesser von ca. 170 nm verwendet. Diese Beads bestehen aus einer Polystyrol-Matrix, in welcher Magnetit-Partikel eingeschlossen sind. Die Oberfläche der Beads wurde mit Biotin funktionalisiert.

Die Gesamtmenge der Flüssigkeit betrug stets 100 $\mu$l. Die Konzentration der magnetisierbaren Beads betrug 1,3 $\cdot 10^{12}$ Beads/ml.

Es wurde eine Referenzmessung gemacht, bei der kein Streptavidin hinzugegeben wurde. Anschließend wurden Messungen mit unterschiedlichen Streptavidin-Konzentrationen gemacht. Streptavidin geht mit Biotin eine Bindung ein. Da Streptavidin vier Biotin-Bindungsstellen besitzt, aggregieren die Beads.

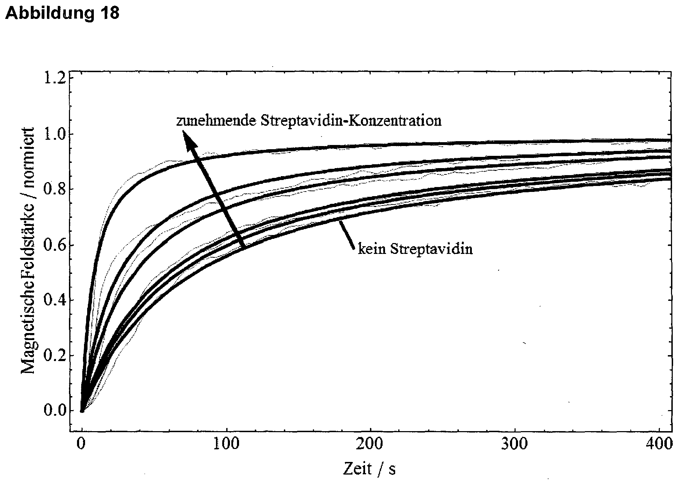

Die Ergebnisse sind in **Abbildung 18** dargestellt:

In Pfeilrichtung nimmt die Streptavidin-Konzentration dabei wie folgt zu: kein Streptavidin; 41,67 nmol/ml; 83,33 nmol/ml; 208,33 nmol/ml; 333,33 nmol/ml; 500 nmol/ml.

Die Originalkurven sind in grau dargestellt. Schwarz überlagert sind angefittete Kurven dargestellt. Es wurde die Langmuir-Gleichung

$$\left|\vec{B}_{Fit}(t)\right| = P \cdot \frac{t/\tau(c)}{1 + t/\tau(c)}$$

verwendet. Die Zeitkonstanten wurden dabei wie folgt bestimmt:

T(0 nmol/ml) = 78,38 s;
T(41,67 nmol/ml) = 67,25 s;
T(83,33 nmol/ml) = 60,23 s;
T(208,33 nmol/ml) = 36,49 s;
T(333,33 nmol/ml) = 26,04 s;
T(500 nmol/ml) = 8,34 s.

**Beispiel 3: Nachweis eine Analyten mit magnetisierbaren und nicht-magnetisierbaren Beads**

Es wurde wieder ein Aufbau verwendet, bei dem die Beads in ein horizontal verlaufendes Feld hineingezogen werden (siehe **Abbildung 16**).

Zur Messung der magnetischen Feldstärke diente der Sensor STJ-220 von Micro Magnetics (www.micromagnetics.com).

Es wurden ebenfalls magnetisierbare Beads der Firma Merck Millipore mit einem Durchmesser von ca. 170 nm verwendet. Die Oberfläche der Beads wurde mit dem Antigen Myeloperoxidase (MPO) funktionalisiert.

Als nicht-magnetisierbare Beads dienten Polystyrol-Beads, ebenfalls der Marke Estapor von Merck Millipore. Diese hatten einen Durchmesser von ca. 1000 nm. Die nicht-magnetisierbaren Beads wurden mit Anti-Human-IgG funktionalisiert.

Die Gesamtmenge der Flüssigkeit betrug jeweils 130 μl. Die Konzentration der magnetisierbaren Beads betrug $7,77 \cdot 10^{11}$ Beads/ml, die Konzentration der nicht-magnetisierbaren Beads betrug $1,4 \cdot 10^{11}$ Beads/ml.

Die magnetisierbaren Beads wurden zunächst mit dem zu untersuchenden Serum vermischt. Nachzuweisende Antikörper gegen MPO (sofern vorhanden) banden an die Antigene auf der Oberfläche der Beads. Anschließend wurden die nicht-magnetisierbaren Beads hinzugegeben. Die hierauf immobiliserten Anti-Human-IgG-s banden an vorhandene Antikörper. Die im Verhältnis kleineren magnetisierbaren Beads wurden so auf den größeren nicht-magnetisierbaren Beads immobilisiert.

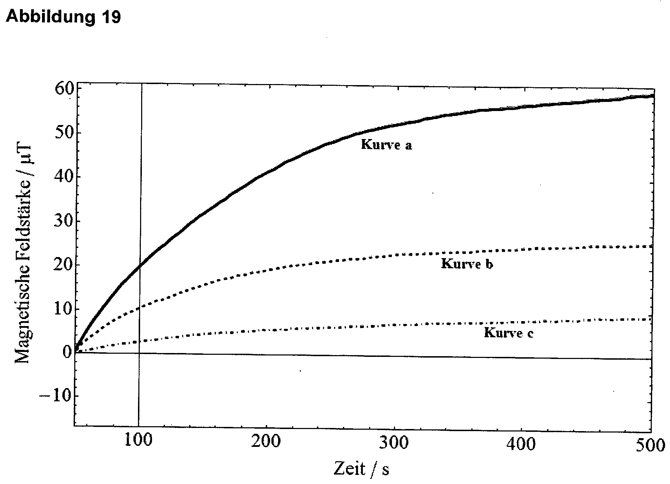

Die Ergebnisse sind in **Abbildung 19** dargestellt. Die durchgezogene Linie zeigt eine Messung ohne vorhandene Antikörper gegen MPO (Kurve a), die Strich-Punkt-Kurve zeigt eine Messung mit hoch-positivem Serum (sehr viele Antikörper gegen MPO, Kurve c), die gestrichelte Kurve zeigt eine Messung mit gegenüber dem hoch-positiven Serum halbierter MPO-Antikörper-Konzentration (Kurve b).

Bei dieser Messung wird die absolute Feldstärke zur Ermittlung der Antikörper-Konzentration verwendet.

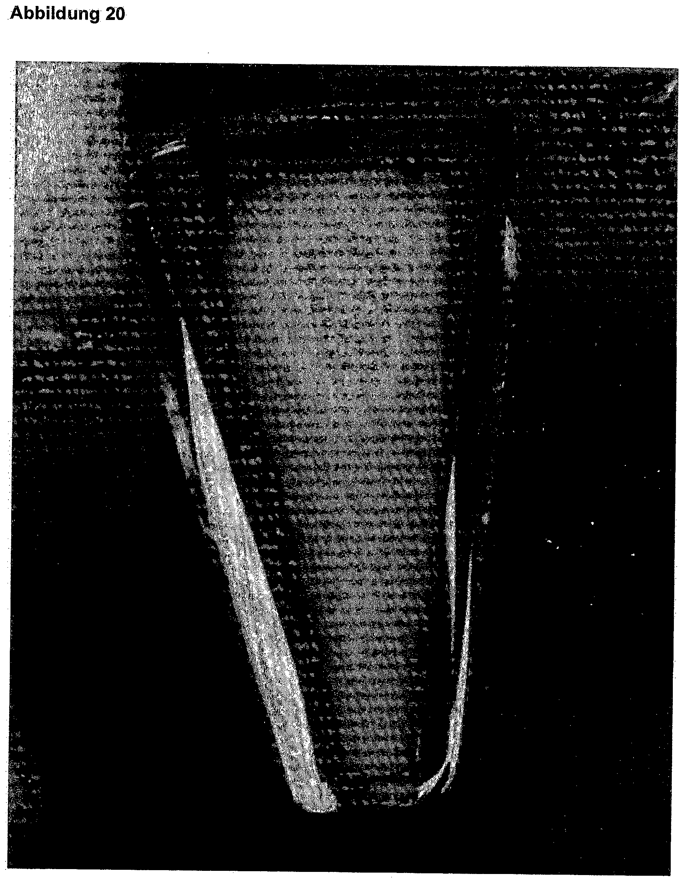

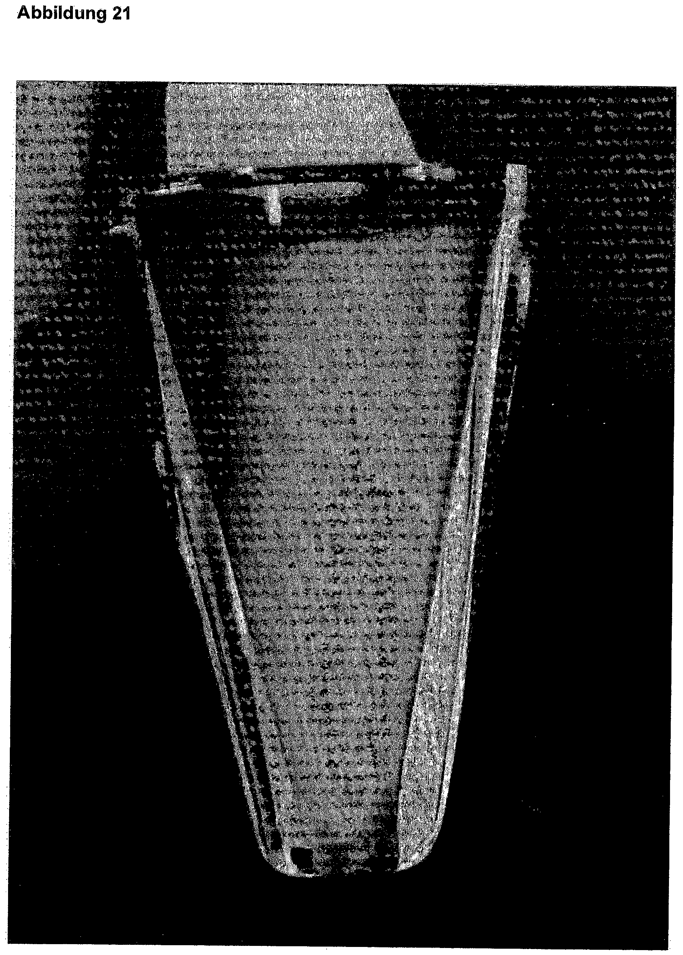

Die **Abbildungen 20** und **21** zeigen die nicht aggregierten bzw. die aggregierten Beads.

**Patentansprüche**

1. Verfahren zum Nachweis eines Analyten in einer Probe, umfassend die Schritte:

   (a) Inkontaktbringen der Probe mit magnetisierbaren Beads und nicht-magnetisierbaren Beads, wobei die Beads mit Bindemolekülen beschichtet sind, und wobei die magnetisierbaren Beads mit Molekülen vom Typ A beschichtet sind und die nicht-magnetisierbaren Beads mit Molekülen vom Typ B beschichtet sind, so dass die Analytmoleküle eine Verbindung zwischen den nicht-magnetisierbaren und den magnetisierbaren Beads herstellen und abhängig vom Vorhandensein und/oder der Konzentration des Analyten in der Probe eine Aggregation der magnetisierbaren Beads erfolgt, und

(b) Bestimmen des Aggregationsgrades der magnetisierbaren Beads durch magnetische Detektion,

**dadurch gekennzeichnet,**

**dass** die Schritte (a) und (b) in einer Testkavität durchgeführt werden, wobei Schritt (b) das Anlegen eines Magnetfeldes umfasst, so dass sich die Testkavität in einem starken stationären Magnetfeld befindet, welches hauptsächlich in horizontaler Richtung verläuft und dessen Betrag nach unten hin zunimmt, wodurch eine Bewegung der magnetisierbaren Beads hervorgerufen wird, so dass sich eine Schicht aus Beads bzw. Bead-Aggregaten ausbildet, die in Richtung der Feldlinien des externen Magnetfelds magnetisiert sind und ein zusätzliches Magnetfeld erzeugen, das unter Verwendung eines Magnetfeldsensors gemessen wird, der so unter der Testkavität positioniert ist, dass die vertikale Komponente des von der Beadschicht erzeugten Magnetfelds gemessen werden kann, wobei der Magnetfeldsensor nicht mittig unter der Testkavität, sondern in N-S-Richtung versetzt ist.

**2.** Verfahren nach einem der vorhergehenden Ansprüche,
**dadurch gekennzeichnet, dass** in Schritt (b) eine Detektion des Aggregationsgrades der Beads eine Bestimmung der Bewegung der magnetisierbaren Beads in einem inhomogenen Magnetfeld umfasst.

**3.** Verfahren nach einem der vorhergehenden Ansprüche,
**dadurch gekennzeichnet,**
**dass** man eine Testkavität verwendet, die ein Filtrationselement enthält, das eine größere Durchlässigkeit für nicht-aggregierte Beads als für Bead-Aggregate aufweist.

**4.** Verfahren nach einem der vorhergehenden Ansprüche,
**dadurch gekennzeichnet,**
**dass** Schritt (b) das Anlegen eines Magnetfeldes unter Verwendung eines Ringmagneten mit Nullfeldpunkten umfasst.

**5.** Verfahren nach einem der vorhergehenden Ansprüche,
**dadurch gekennzeichnet,**
**dass** Schritt (b) das Anlegen eines Magnetfeldes mit zeitlichen Unterbrechungen umfasst.

## Claims

**1.** Method for detecting an analyte in a sample, comprising the following steps:

(a) bringing the sample into contact with magnetisable beads and non-magnetisable beads, the beads being coated with binding molecules, and the magnetisable beads being coated with type A molecules and the non-magnetisable beads being coated with type B molecules, such that the analyte molecules establish a bond between the non-magnetisable and the magnetisable beads, and the magnetisable beads aggregate depending on the presence and/or concentration of the analyte in the sample, and

(b) determining the degree of aggregation of the magnetisable beads by magnetic detection,

**characterised in that**

steps (a) and (b) are carried out in a test well, step (b) comprising applying a magnetic field, such that the test well is located in a strong, stationary magnetic field which predominantly extends in the horizontal direction and the strength of which increases downwards, as a result of which the magnetisable beads are caused to move such that a layer of beads or bead aggregates forms, which beads or bead aggregates are magnetised in the direction of the field lines of the external magnetic field and generate an additional magnetic field which is measured using a magnetic field sensor that is positioned below the test well in such a way that the vertical component of the magnetic field generated by the bead layer can be measured, the magnetic field sensor not being centrally offset below the test cavity, but rather offset in the N-S direction.

**2.** Method according to any of the preceding claims, **characterised in that** in step (b), detecting the degree of aggregation of the beads comprises determining the movement of the magnetisable beads in an inhomogeneous magnetic field.

**3.** Method according to either of the preceding claims, **characterised in that** a test well is used which contains a filtration element that is more permeable to non-aggregated beads than to bead aggregates.

4. Method according to any of the preceding claims, **characterised in that** step (b) comprises applying a magnetic field by using a ring magnet having zero-field points.

5. Method according to any of the preceding claims, **characterised in that** step (b) comprises applying an intermittent magnetic field.


**Revendications**

1. Procédé servant à démontrer la présence d'un analyte dans un échantillon, comprenant les étapes suivantes :

(a) la mise en contact de l'échantillon avec des billes magnétisables et des billes non magnétisables, dans lequel les billes sont recouvertes de molécules de liaison, et dans lequel les billes magnétisables sont recouvertes de molécules du type A et les billes non magnétisables sont recouvertes de molécules du type B de sorte que les molécules d'analytes établissent une liaison entre les billes non magnétisables et les billes magnétisables et qu'une agrégation des billes magnétisables est effectuée en fonction de la présence et/ou de la concentration de l'analyte dans l'échantillon, et
(b) la détermination du degré d'agrégation des billes magnétisables par une détection magnétique,

**caractérisé en ce**
**que** les étapes (a) et (b) sont effectuées dans une cavité de test, dans lequel l'étape (b) comprend l'application d'un champ magnétique de sorte que la cavité de test se trouve dans un champ magnétique stationnaire intense, qui s'étend principalement dans la direction horizontale et dont la valeur augmente vers le bas ce qui permet de provoquer un déplacement des billes magnétisables, de sorte qu'une couche se forme à partir de billes ou d'agrégats de billes, qui sont magnétisés en direction des lignes de champ du champ magnétique externe et qui produisent un champ magnétique supplémentaire, qui est mesuré en utilisant un capteur de champ magnétique, qui est positionné de telle manière sous la cavité de test que le composant vertical du champ magnétique produit par la couche de billes peut être mesuré, dans lequel le capteur de champ magnétique est décalé non pas au centre sous la cavité de test, mais dans la direction N-S.

2. Procédé selon l'une quelconque des revendications précédentes,
**caractérisé en ce qu'**à l'étape (b), une détection du degré d'agrégation des billes comprend une détermination du déplacement des billes magnétisables dans un champ magnétique non homogène.

3. Procédé selon l'une quelconque des revendications précédentes,
**caractérisé en ce**
**qu'**on utilise une cavité de test, qui contient un élément de filtration, qui présente une perméabilité plus importante pour des billes non agrégées que pour des agrégats de billes.

4. Procédé selon l'une quelconque des revendications précédentes,
**caractérisé en ce**
**que** l'étape (b) comprend l'application d'un champ magnétique utilisant un aimant annulaire présentant des points de champ nul.

5. Procédé selon l'une quelconque des revendications précédentes,
**caractérisé en ce**
**que** l'étape (b) comprend l'application d'un champ magnétique présentant des interruptions dans le temps.

**Abbildung 1**

Beads, welche mit dem
Molekül des Typs A beschichtet sind

Suspendiertes
Bead

Analyt C

Analyt C
in Lösung

Test-Kavität

**Abbildung 2**

Bead, welches mit Molekülen des Typs A beschichtet ist

Bead, welches mit Molekülen des Typs B beschichtet ist

Analyt D

Analyt D in Lösung

Suspendiertes Bead

Test-Kavität

**Abbildung 3**

Test-Kavität

Bead, welches mit Molekülen des Typs A beschichtet ist

Bead, welches mit Molekülen des Typs B beschichtet ist

Analyt E

Verbindungsmolekül D

Gelöste Moleküle

Suspendiertes Bead

**Abbildung 4**

Bead, welches mit dem Molekül des Typs A beschichtet ist

Bead, welches mit dem Analyt C beschichtet ist

Suspendiertes Bead

Test-Kavität

**Abbildung 5**

**Abbildung 6**

Beads

Bead-Aggregate

Sieb

Bead-Aggregate

Bewegungsrichtung der Beads bzw. der Bead-Aggregate

Magnet

**Abbildung 7**

Feldlinien

Nullfeld-Punkt

Ringmagnet

## Abbildung 8

Test-Kavität

Richtung des Magnetfeld-Gradienten

Bead-Aggregate

Position des Magnetfeld-Sensors

**Abbildung 9**

Feldlinien

Spulen-Wicklungen

**Abbildung 10**

Test-Kavität

Richtung des Magnetfeld-Gradienten

Bead-Aggregate

Position des Magnetfeld-Sensors

Spulenwicklungen

**Abbildung 11**

Test-Kavität

Richtung des Magnetfeld-Gradienten

Bead-Aggregate

Position des Magnetfeld-Sensors

**Abbildung 12**

**Abbildung 13**

Test-Kavität

Magnetisierbare Beads, welche mit Molekülen des Typs A beschichtet sind

D

Analyt D in Lösung

Analyt D

Nicht-magnetisierbares Bead, welches mit Molekülen des Typs B beschichtet ist

Suspendiertes Bead

**Abbildung 14**

**Abbildung 15**

**Abbildung 16**

Test-Kavität

Linien gleicher absoluter
magnetischer Feldstärke

S N · S N

Permanentmagnet    Bead-Layer    Magnetfeld-Sensor

**Abbildung 17**

Magnetisierter Bead-Layer

Magnetfeld-Sensor

Feldlinien des durch den Bead-Layer erzeugten Magnetfeldes

**Abbildung 18**

**Abbildung 19**

**Abbildung 20**

**Abbildung 21**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6294342 B1